# EUROPÄISCHE PATENTSCHRIFT

(11) **EP 1 309 578 B1**
(45) Veröffentlichungstag und Bekanntmachung des Hinweises auf die Patenterteilung: **18.02.2004**
(21) Anmeldenummer: 01976093.3
(22) Anmeldetag: 16.08.2001
(51) Int. Cl.: C07D 285/34

(54) **VERFAHREN ZUR HERSTELLUNG EINER TEILCHENFÖRMIGEN ZUBEREITUNG VON TETRAHYDRO- 3,5-DIMETHYL-1,3,5-THIADIAZIN-2-THION**
METHOD FOR THE PRODUCTION OF A PARTICLE-CONTAINING PREPARATION OF TETRAHYDRO- 3,5-DIMETHYL-1,3,5-THIADIAZIN-2-THIONE
PROCEDE DE PRODUCTION D'UNE PREPARATION SOUS FORME DE PARTICULES DE TETRAHYDRO- 3,5-DIMETHYL-1,3,5-THIADIAZIAN-2-THION

(30) Priorität: 17.08.2000 DE 10040194
(43) Veröffentlichungstag der Anmeldung: 14.05.2003
(73) Patentinhaber: BASF AKTIENGESELLSCHAFT, 67056 Ludwigshafen (DE)
(72) Erfinder: ISAK, Heinz, 67459 Böhl-Iggelheim (DE); SENDHOFF, Norbert, 67269 Grünstadt (DE); SCHÜTZ, Franz, 67435 Neustadt (DE); THERRE, Jörg, 67551 Worms (DE); DRÖGEMÜLLER, Michael, 29525 Uelzen (DE); FRANKE, Dirk, 67134 Birkenheide (DE); MUNZINGER, Manfred, 67246 Dirmstein (DE); WECK, Alexander, 67251 Freinsheim (DE); BURKART, Kirsten, 67069 Ludwigshafen (DE)
(74) Vertreter: Pohl, Michael, Dr.
(86) Internationale Anmeldenummer: PCT/EP2001/009471
(87) Internationale Veröffentlichungsnummer: WO 2002/014296

(56) Entgegenhaltungen:
- WO-A-93/13085
- DD-A- 289 055
- FR-A- 1 554 038
- DATABASE WPI Section Ch, Week 198503 Derwent Publications Ltd., London, GB; Class C02, AN 1985-014717 XP002183831 & JP 59 210073 A (SANSHIN KAGAKU KOGYO CO) , 28. November 1984 (1984-11-28)
- DATABASE WPI Section Ch, Week 198007 Derwent Publications Ltd., London, GB; Class C02, AN 1980-11991C XP002183832 & JP 55 002620 A (BASF JAPAN ET AL), 10. Januar 1980 (1980-01-10)

## Beschreibung

Die vorliegende Erfindung betrifft ein Verfahren zur Herstellung einer teilchenförmigen Zubereitung von Tetrahydro-3,5-dimethyl-1,3,5-thiadiazin-2-thion durch Vereinigen einer Methylammonium-N-methyldithiocarbamat enthaltenden ersten wässrigen Lösung mit einer Formaldehyd enthaltenden zweiten wässrigen Lösung und Abtrennen und Trocknen des gebildeten Feststoffs.

Tetrahydro-3,5-dimethyl-1,3,5-thiadiazin-2-thion, das auch unter der Kurzbezeichnung Dazomet bekannt ist, wird in der Landwirtschaft und Gärtnerei zur Bodenentseuchung, d.h. zur Bekämpfung von Nematoden, keimenden Pflanzen und Bodenpilzen eingesetzt (vgl. US-A 2,838,389). Die Wirkung beruht auf der langsamen Freisetzung von Methylisothiocyanat im Boden durch hydrolytische und/oder enzymatische Zersetzung von Tetrahydro-3,5-dimethyl-1,3,5-thiadiazin-2-thion.

Um die Bildung potentiell gesundheitsschädlicher Staubnebel beim Abfüllen, bei der Handhabung und/oder bei der Ausbringung des Wirkstoffs zu verhindern, ist eine teilchenförmige Zubereitung von Tetrahydro-3,5-dimethyl-1,3,5-thiadiazin-2-thion mit möglichst geringem Feinanteil mit Teilchengrößen von weniger als 100 µm erwünscht. Um andererseits eine hinreichend schnelle Zersetzung des Wirkstoffs im Boden zu gewährleisten, sollte die teilchenförmige Zubereitung auch keinen wesentlichen Grobanteil mit Teilchengrößen von mehr als 400 µm aufweisen. Die bekannten Herstellungsverfahren von Tetrahydro-3,5-dimethyl-1,3,5-thiadiazin-2-thion gestatten die Herstellung teilchenförmiger Zubereitungen von Tetrahydro-3,5-dimethyl-1,3,5-thiadiazin-2-thion mit einer hinreichend homogenen Teilchengrößenverteilung nur unzureichend.

Die WO 93/13085 beschreibt ein Verfahren zur Herstellung eines Granulats von Tetrahydro-3,5-dimethyl-1,3,5-thiadiazin-2-thion durch Umsetzung des Methylammoniumsalzes der N-Methyldithiocarbaminsäure mit Formaldehyd in Gegenwart eines Diaminoalkylens. Das Diaminoalkylen führt unter Reaktionsbedingungen zur Bildung von Produkten, die als Kristallisationsstörer wirken und mit den Kristalliten des Wirkstoffs ein ungeordnetes Konglomerat bilden. Die WO 93/13085 empfiehlt, die Lösung des Methylammonium-N-methyldithiocarbamats zu einer vorgelegten wässrigen Formaldehydlösung zu geben. Es hat sich gezeigt, dass nach dem bekannten Verfahren reproduzierbare Teilchengrößenverteilungen nur bei genauer Einhaltung einer großen Zahl von Parametern, einschließlich der Geschwindigkeit der Zugabe der Reaktionspartner, der Stärke der Durchmischung der Reaktionspartner, der Dauer der Durchmischung usw., erhalten werden. Dies erschwert die Herstellung eines Produkts mit gleichbleibenden Eigenschaften und ein flexibles Reagieren auf Nachfrageschwankungen.

Der vorliegenden Erfindung liegt daher die Aufgabe zugrunde, ein Verfahren zur Herstellung einer teilchenförmigen Zubereitung von Tetrahydro-3,5-dimethyl-1,3,5-thiadiazin-2-thion bereit zu stellen, das bei einfacher Verfahrensführung Zubereitungen mit einer engen Teilchengrößenverteilung, insbesondere mit einem verringerten Feinanteil einer Teilchengröße von weniger als 100 µm, liefert.

Erfindungsgemäß wird diese Aufgabe gelöst durch ein Verfahren zur Herstellung einer teilchenförmigen Zubereitung von Tetrahydro-3,5-dimethyl-1,3,5-thiadiazin-2-thion durch Vereinigen einer Methylammonium-N-methyldithiocarbamat enthaltenden ersten wässrigen Lösung mit einer Formaldehyd enthaltenden zweiten wässrigen Lösung und Abtrennen und Trocknen des gebildeten Feststoffs, das dadurch gekennzeichnet ist, dass man die erste und die zweite wässrige Lösung so vereinigt, dass das Verhältnis der Konzentrationen von Dithiocarbamat-Funktionen und Formaldehyd im Reaktionsgemisch zeitlich im Wesentlichen konstant ist und aus der erhaltenen Suspension den Feststoff abtrennt.

Die Erfindung betrifft außerdem dieses Verfahren zur Herstellung einer teilchenförmigen agrotechnischen Zubereitung mit einem Gehalt an Tetrahydro-3,5-dimethyl-1,3,5-thiadiazin-2-thion von wenigstens 95 Gew.-% und einer solchen Teilchengrößenverteilung, dass weniger als 7 Gew.-%, vorzugsweise weniger als 3 Gew.-%, der Teilchen einen Teilchendurchmesser von weniger als 100 µm, mehr als 50 Gew.-% der Teilchen einen Teilchendurchmesser von weniger als 200 µm, mehr als 90 Gew.-% der Teilchen einen Teilchendurchmesser von weniger als 300 µm und mehr als 95 Gew.-% der Teilchen einen Teilchendurchmesser von weniger als 400 µm aufweisen.

Die nach dem erfindungsgemäßen Verfahren erhaltene teilchenförmige Zubereitung weist vorzugsweise eine Schüttdichte von 0,4 bis 0,8 kg/l, insbesondere 0,6 bis 0,7 kg/l, auf.

Es wurde gefunden, dass enge Teilchengrößeverteilungen dann erhalten werden können, wenn die erste und die zweite wässrige Lösung so vereinigt werden, dass über die Dauer der Umsetzung das Verhältnis der molaren Konzentration von Dithiocarbamat-Funktion und Formaldehyd im Reaktionsgemisch im Wesentlichen konstant ist.

Das erfindungsgemäße Verfahren unterscheidet sich damit wesentlich von den bekannten Verfahren, bei denen ein Reaktionspartner, in der Regel die wässrige Formaldehydlösung, vorgelegt wird und der andere Reaktionspartner über eine Zeitspanne zudosiert wird. Ersichtlich liegt bei den bekannten Verfahren der vorgelegte Reaktionspartner zu Beginn der Zudosierung in einem vielfachen molaren Überschuß vor. Über die Dauer der Dosierung nimmt das Verhältnis des vorgelegten Reaktionspartners zum zudosierten Reaktionspartner dann fortlaufend ab.

Das erfindungsgemäße Verfahren kann semikontinuierlich oder kontinuierlich durchgeführt werden. Zweckmäßigerweise führt man dazu in einen Reaktionsraum pro Zeiteinheit im Wesentlichen stöchiometrisch äquivalente Mengen der ersten wässrigen Lösung, gerechnet als Dithiocarbamat-Funktionen, und der zweiten wässrigen Lösung, gerechnet als Formaldehyd, d.h. im Wesentlichen die doppelte molare Menge an Formaldehyd ein. Als "im Wesentlichen stöchiometrisch äquivalent" wird eine Menge angesehen, die innerhalb von 20%, vorzugsweise innerhalb von 10%, der stöchiometrisch erforderlichen Menge des jeweiligen Reaktionspartners liegt. Man kann auch eine größere als die stöchiometrisch erforderliche Menge eines Reaktionspartners einbringen, wenn man durch geeignete Maßnahmen die Akkumulation des im Überschuß eingesetzten Reaktionspartners im Reaktionsgemisch verhindert. Die Akkumulation kann z.B. durch kontinuierliches Ausschleusen des Überschusses verhindert werden, wozu beispielsweise ein Teil der bei der Abtrennung des Produkts vom Reaktionsgemisch erhaltenen Mutterlauge kontinuierlich verworfen wird, wie dies nachstehend näher erläutert ist.

Für die kontinuierliche Durchführung des Reaktionsverfahrens sind gängige Reaktoren geeignet, wie insbesondere ein kontinuierlicher Rührkesselreaktor oder eine Rührkesselkaskade. Zweckmäßigerweise wird für eine gute Durchmischung der Reaktionspartner im Reaktionsraum gesorgt. Das Einführen der ersten und/oder zweiten wässrigen Reaktionslösung kann z.B. so erfolgen, dass aus dem Reaktionsraum kontinuierlich eine Teilmenge des Reaktiongemisches entnommen wird, mit der ersten und/oder zweiten wässrigen Reaktionslösung vermischt wird und in den Reaktionsraum zurückgeführt wird. Die Entnahme, das Vermischen und Zurückführen erfolgen z.B. durch Umpumpen des Reaktionsgemisches über eine Dosierungs- und Mischstrecke, in der die erste und/oder zweite Lösung eingespeist werden. Anstelle von Rührkesselreaktoren oder -kaskaden kann man auch Rohrreaktoren verwenden, die gegebenenfalls mit Einbauten, wie statischen Mischern, versehen sind.

Beim Vereinigen der wässrigen Lösungen bildet sich schwerlösliches Tetrahydro-3,5-dimethyl-1,3,5-thiadiazin-2-thion in Form einer wässrigen Suspension. Bei kontinuierlicher Durchführung des erfindungsgemäßen Verfahrens wird kontinuierlich Suspension aus dem Reaktionsraum bzw. - bei Verwendung einer Reaktorkaskade - aus dem letzten Reaktor der Kaskade abgezogen. Aus der erhaltenen Suspension wird der Feststoff nach üblichen Verfahren, z.B. durch Filtration oder Zentrifugation, abgetrennt. Geeignete Vorrichtungen, wie Drucknutschen, Vakuumbandfilter, Drehtrommelfilter und Zentrifugen sind dem Fachmann bekannt. Die zurückbleibende Mutterlauge kann ganz oder teilweise zurückgeführt oder aus dem Verfahren ausgeschleust werden. Durch Ausleiten wenigstens eines Teils der Mutterlauge kann eine Anhäufung von Verunreinigungen oder im Überschuß eingesetzter Reaktionspartner im System verhindert werden.

Der von der Mutterlauge abgetrennte Feststoff kann z.B. mit kaltem oder warmem Wasser, gewaschen werden. Hierzu kann der Feststoff mit dem Waschmedium aufgeschlämmt und anschließend abgetrennt werden.

Der von der Mutterlauge abgetrennte und gegebenenfalls gewaschene Feststoff kann dann nach üblichen Verfahren getrocknet werden. Hierzu sind Stromtrockner oder Wirbelbetten geeignet.

Im Einzelfall kann es vorteilhaft sein, den nach der Abtrennung von der Mutterlauge erhaltenen feuchten Kuchen mit bereits getrocknetem Material zu vermischen und das Gemisch der weiteren Trocknung zu unterziehen, um auf diese Weise ein Verbacken beim Trocknen zu verhindern. Bei kontinuierlicher Verfahrensführung kann dies durch die Rückführung eines Teils des getrockneten Materials erreicht werden.

Vorzugsweise vereinigt man die erste und zweite wässrige Lösung in Gegenwart von Impfkristallen von Tetrahydro-3,5-dimethyl-1,3,5-thiadiazin-2-thion. Durch die Zugabe von Impfkristallen kann zusätzlich Einfluß auf die Teilchengrößenverteilung und/oder die Schüttdichte der erhaltenen teilchenförmigen Zubereitung genommen werden. Als Impfkristalle verwendet man fein verteiltes Tetrahydro-3,5-dimethyl-1,3,5-thiadiazin-2-thion z.B. in einer Menge von 1,5 Mol-% bis 10 Mol-%, vorzugsweise 2,5 Mol-% bis 7,5 Mol-%, insbesondere 3 Mol-% bis 6 Mol-%, bezogen auf das eingesetzte Methylammonium-N-methyldithiocarbamat. Vorzugsweise finden Impfkristalle mit einer Korngröße von weniger als 100 µm Verwendung. Üblicherweise sollen die Korngrößen der Impfkristalle zu 90% zwischen 50 und 5 µm liegen. Insbesondere bevorzugt ist eine Korngrößenverteilung der Impfkristalle, in der die Teilchen zu 100% kleiner als 100 µm sind, zu etwa 90% zwischen 50 und 1 µm liegen und zu etwa 10% kleiner als 5 µm sind.

Um eine möglichst gleichmäßige Verteilung der Impfkristalle im Reaktionsgemisch zu erreichen, setzt man die Impfkristalle dem Reaktionsgemisch vorzugsweise in Form einer wässrigen Suspension zu.

Impfkristalle einer gewünschten Korngröße können durch Zerkleinern, z.B. durch Mahlung, von zuvor hergestelltem Tetrahydro-3,5-dimethyl-1,3,5-thiadiazin-2-thion erhalten werden. In einer möglichen Ausführungsform der Erfindung werden Impfkristalle geeigneter Größe dadurch erhalten, dass der dem Reaktionsraum entnommene teilchenförmige Feststoff einer Größenklassierung unterworfen wird, wobei ein Feingutanteil und ein Grobgutanteil gewonnen wird und der Feingutanteil als Impfkristalle in den Reaktionsraum zurückgeführt und der Grobgutanteil als Produkt aus dem Verfahren ausgeleitet wird. Die Größenklassierung kann sowohl in suspendiertem als auch in trockenem Zustand erfolgen. Zur Klassierung in trockenem Zustand wird der Feststoff zuvor von der Mutterlauge getrennt und getrocknet. Der gewonnene Feingutanteil kann zur Rückführung in den Reaktionsraum in einem wässrigem Medium suspendiert werden. Geeignete Vorrichtungen zur Größenklassierung sind z.B. Hydrozyklone und Naßsiebe für die Klassierung suspendierter Teilchen sowie Zyklone, Siebe oder Sichter für die Klassierung getrockneter Teilchen.

Zur Herstellung der ersten wässrigen Lösung geht man im Allgemeinen so vor, dass man zunächst eine wässrige Lösung von Methylamin, gegebenenfalls unter Mitverwendung eines Alkylendiamins wie unten beschrieben, mit Schwefelkohlenstoff versetzt. Die Umsetzung der wässrigen Methylaminlösung, gegebenenfalls unter Mitverwendung eines Alkylendiamins, mit Schwefelkohlenstoff kann kontinuierlich, semikontinuierlich oder absatzweise erfolgen. Die kontinuierliche Umsetzung kann in beliebigen dazu geeigneten Reaktoren, z.B. Reaktionskolonnen oder Strahlschlaufen oder vorzugsweise in Rührkesseln durchgeführt werden. Um eine möglichst vollständige Umsetzung zu erreichen, empfiehlt sich eine Reaktorkaskade aus wenigstens einem Hauptreaktor und einem Nachreaktor.

vorzugsweise verwendet man zur Herstellung der ersten wässrigen Lösung einen Überschuss an Schwefelkohlenstoff. Nicht umgesetzter Schwefelkohlenstoff scheidet sich als spezifisch schwerere Phase von der wässrigen Lösung des Dithiocarbamats ab. Es hat sich als vorteilhaft erwiesen, den nicht umgesetzten Schwefelkohlenstoff auf weniger als 0,5 Gew.-%, insbesondere weniger als 0,3 Gew.-%, besonders bevorzugt weniger als 0,1 Gew.-%, bezogen auf die erste wässrige Lösung zu entfernen, bevor diese mit der zweiten wässrigen Lösung vereinigt wird. Größere Mengen überschüssigen Schwefelkohlenstoffs neigen zur Bildung feindispergierter Tröpfchen, die bei der anschließenden Umsetzung mit der wässrigen Formaldehydlösung den Fällungsprozess durch Flotation stören und unerwünschterweise zur vermehrten Bildung von ultrafeinen Teilchen einer Größe von weniger als z.B. 10 µm führen können. Die Abtrennung des nicht umgesetzten Schwefelkohlenstoffs kann z.B. durch Absetzenlassen und anschließende Phasentrennung erreicht werden. Zum Absetzenlassen kann man das Gemisch aus wässriger Carbamatlösung und nicht umgesetztem Schwefelkohlenstoff in eine Beruhigungszone leiten. Dies geschieht zweckmäßigerweise in einem liegenden, kontinuierlich betriebenem Phasentrenngefäß, das mit geringer Strömungsgeschwindigkeit durchflossen wird. Bedingt durch den Dichteunterschied der Phasen trennt sich die Emulsion im Erdschwerefeld, so dass beide Phasen in zusammenhängender Form und weitgehend fremdphasenfrei übereinander geschichtet vorliegen.

Eine weitgehende Entfernung der Schwefelkohlenstoffphase erfordert nachteiligerweise sehr lange Absetzzeiten. Um eine schnellere Phasentrennung zu erreichen benutzt man mit Vorteil eine oder mehrere Koaleszenzstufen mit -integrierter oder nachgeschalteter Phasentrenneinrichtung. Geeignet sind im Allgemeinen Abscheider mit Koaleszenzeinbauten, wie Füllkörpern, Koaleszenzflächen oder feinporigen Elementen, über die oder durch die die zu trennende Emulsion fließen muß. Gegebenenfalls entfernt man zuerst die Hauptmenge des nicht umgesetzten Schwefelkohlenstoffs und leitet die noch feindispergierte Schwefelkohlenstofftröpfchen enthaltende wässrige Lösung durch eine Apparatur mit koaleszenzfördernden Einbauten und trennt die koaleszierte Schwefelkohlenstoffphase ab.

Bei den Koaleszenzflächeneinbauten handelt es sich in der Regel um Plattenpakete mit gewellten oder schräggestellten Flächen, an denen sich dispergierte Tröpfchen anlagern und zunächst einen Film ausbilden. Wenn dieser Film die Einzelplatte umschließt und dick genug ist, dann bilden sich große Tropfen der dispergierten Phase an der Plattenkante aus und fallen nach unten. Sie bilden dann eine Schicht im Abscheider, die mechanisch leicht abzutrennen ist. Bei feinporigen Einbauten zwingt die innere Struktur der Elemente die feindispergierten Tropfen zum Kontakt mit der inneren Oberfläche, die dann einen Film bilden und als vereinigte größere Tropfen die hohle Struktur der feinporigen Elemente verlassen. Als Füllkörper sind üblicherweise bei der Destillation verwendete Füllkörper geeignet. Vorzugsweise leitet man die zu trennende Emulsion durch eine Füllkörperschüttung. Durch Benetzung der großen Füllkörperoberfläche kommt es zur Oberflächenkoaleszenz und gleichzeitig durch Tropfenbewegung zu Tropfen-Tropfen-Koaleszenz.

Bewährt haben sich poröse Einbauten in Form von Filterkerzen, die z.B. aus porösem Polypropylen bestehen. Eine mittlere Porengröße der porösen Einbauten von weniger als 50 µm ist besonders geeignet.

Da sowohl die Reaktion von Schwefelkohlenstoff mit Methylamin als auch die Reaktion von Methylammonium-N-methyldithiocarbamat mit Formaldehyd exotherm verläuft, Zwischenprodukt und Produkt hingegen thermisch labil sind, ist bei der Umsetzung eine Reaktionsführung unter Kühlung empfehlenswert. Im Allgemeinen verlaufen die Umsetzungen oberhalb von 10°C mit ausreichender Geschwindigkeit, während es oberhalb von 50°C vermehrt zur Bildung unerwünschter Nebenprodukte kommt. Daher werden die Umsetzungen üblicherweise bei einer Temperatur von 20 bis 40°C durchgeführt.

In bevorzugten Ausführungsformen des erfindungsgemäßen Verfahrens enthält die erste wässrige Lösung neben dem Methylammonium-N-methylthiocarbamat wenigstens ein Alkylendiamin und/oder Umsetzungsprodukte davon mit Schwefelkohlenstoff. Zweckmäßigerweise enthält die erste wässrige Lösung 0,1 bis 10 Mol-%, vorzugsweise 0,2 bis 5 Mol-%, insbesondere 0,5 bis 1,5 Mol-%, Alkylendiamin, bezogen auf die Menge an zugrunde liegendem Methylamin. Geeignete Alkylendiamine entsprechen der Formel I

R¹-NH-A-NH-R² (I)

worin R¹ und R² unabhängig voneinander für Wasserstoff oder C₁-C₄-Alkyl stehen, und A für geradkettiges oder verzweigtes C₂-C₈-Alkylen, vorzugsweise 1,2-Ethylen, 1,2-Propylen, 1,3-Propylen oder 1,4-Butylen, steht.

Bevorzugte Alkylendiamine sind die in der WO 93/13085 genannten. Hiervon sind Ethylendiamin, 1-(N-Methylamino)-2-aminoethan, 1,2-Di(N-methylamino)ethan, 1,2-Diaminopropan, 1,2-Di(N-methylamino)propan und 1-(N-Methylamino)-2-aminopropan bevorzugt. Ethylendiamin ist besonders bevorzugt. Es können sowohl die reinen Verbindungen als auch Gemische dieser Verbindungen verwendet werden können.

Die Verwendung von Alkylendiaminen mit zwei primären Aminogruppen, z.B. solche der obigen Formel I, worin R¹ = R² = H, insbesondere Ethylendiamin, ist bevorzugt. Es hat sich gezeigt, dass besonders vorteilhafte Teilchengrößenverteilungen erhalten werden, wenn das Alkylendiamin und seine möglichen Reaktionsprodukte mit Schwefelkohlenstoff, d.h. sein Umsetzungsprodukt mit 1 Mol Schwefelkohlenstoff (N-Aminoalkyldithiocarbamat) und sein Umsetzungsprodukt mit 2 Mol Schwefelkohlenstoff (Alkylen-N,N'-bis(dithiocarbamat)), in einem bestimmten molaren Verhältnis zueinander in der ersten wässrigen Lösung vorliegen. Vorzugsweise enthält die erste wässrige Lösung Alkylendiamin, N-Aminoalkyldithiocarbamat und Alkylen-N,N'-bis(dithiocarbamat) in einem molaren Verhältnis von 1:0,5:0,5 bis 1:10:10, insbesondere von 1:1:1 bis 1:10:6. Die freien Aminofunktionen des Alkylendiamins bzw. seines Umsetzungsproduktes mit 1 Mol Schwefelkohlenstoff können dabei protoniert vorliegen; die Dithiocarbamat-Funktionen liegen in der Regel als N-Methylammoniumsalz oder zusammen mit einer im gleichen Molekül vorliegenden Ammoniumgruppe als inneres Salz vor. Das molare Verhältnis von Alkylendiamin und seiner Umsetzungsprodukte mit Schwefelkohlenstoff wird vorzugsweise mittelbar durch Analyse der nach Umsetzung der ersten und der zweiten wässrigen Lösung erhaltenen Produkte bestimmt. So reagiert ein Alkylendiamin der Formel I mit R¹ = R² = H mit 2 Mol N-Methyldithiocarbamat und 4 Mol Formaldehyd zu (1), N-Aminoalkyldithiocarbamat mit 1 Mol N-Methyldithiocarbamat, 1 Mol Methylammoniumionen und 4 Mol Formaldehyd zu (2) und Alkylen-N,N'-bis(dithiocarbamat) mit 2 Mol N-Methylammoniumionen und 4 Mol Formaldehyd zu (3).

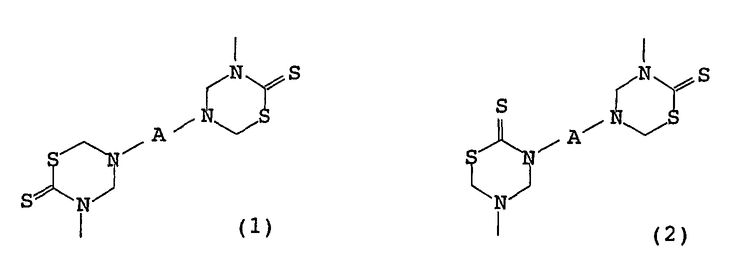

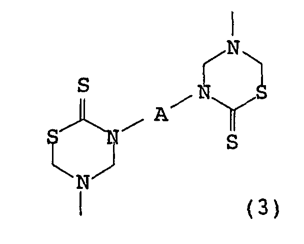

Die Produkte (1), (2) und (3) können geeigneterweise in der erhaltenen Zubereitung (neben der Hauptkomponente Tetrahydro-3,5-dimethyl-1,3,5-thiadiazin-2-thion) durch Hochleistungsflüssigchromatographie getrennt und quantitativ bestimmt werden.

Das molare Verhältnis von Alkylendiamin und seinen Umsetzungsprodukten mit Schwefelkohlenstoff in der ersten wässrigen Lösung kann durch Variation der Reihenfolge der Vereinigung von Methylamin, Schwefelkohlenstoff und Alkylendiamin und/oder der Verweilzeit vor Zugabe eines weiteren Reaktionspartners gesteuert werden. So ist es bevorzugt, zur Herstellung der ersten wässrigen Lösung zuerst eine wässrige Lösung von Methylamin mit Schwefelkohlenstoff umzusetzen und zu der erhaltenen Lösung das Alkylendiamin zu geben. Vorzugsweise ist zum Zeitpunkt der Zugabe von Alkylendiamin die Umsetzung zwischen Methylamin und Schwefelkohlenstoff zu 60 bis 95% fortgeschritten. Die Umsetzung zwischen Methylamin und Schwefelkohlenstoff kann z.B. durch Stichprobennahme, Überwachen des pH-Wertes oder Überwachen der Reaktionsenthalpie verfolgt werden. Bei Verwendung einer Reaktorkaskade zur Herstellung der ersten wässrigen Lösung wird das Alkylendiamin vorzugsweise in den Nachreaktor dosiert, um das angesprochene Verhältnis von Alkylendiamin und seiner Umsetzungsprodukte mit Schwefelkohlenstoff einzustellen.

Es hat sich als vorteilhaft erwiesen, die Umsetzung von erster und zweiter wässriger Lösung in Gegenwart geringer Elektrolytmengen durchzuführen. Dies erreicht man zweckmäßigerweise, indem man zur Herstellung der ersten wässrigen Lösung oder zum Verdünnen des Reaktionsgemisches kein entsalztes Wasser, sondern Leitungsoder Flußwasser verwendet. Vermutlich verhindert die Gegenwart geringer Mengen Elektrolyt eine elektrostatische Aufladung und Agglomeration der gefällten Teilchen. Im Allgemeinen sind Elektrolytmengen geeignet, die einer Leitfähigkeit von 500 bis 1000 µS/cm entsprechen.

Bei Ausübung des erfindungsgemäßen Verfahrens und/oder der Zubereitung der Methylammonium-N-methyldithiocarbamatlösung fällt in der Regel mit Schwefelkohlenstoff belastete Abluft an, die aufgrund behördlicher Regelungen etc. nicht ohne weiteres an die Umgebung abgegeben werden kann. Die mit Schwefelkohlenstoff beladene Abluft kann durch Adsorption an geeignete Adsorbentien, wie Aktivkohle, oder Waschen mit basischen Flüssigkeiten, z.B. wässrigem Natriumhydroxid oder primären, sekundären oder tertiären Aminen, von Schwefelkohlenstoff befreit werden. In einer bevorzugten Ausführungsform führt man die Abgaswäsche mit einer wässrigen Methylaminlösung durch, wobei sich unter weitgehender Abreicherung der Abluft von Schwefelkohlenstoff Methylammonium-N-methyldithiocarbamat bildet. Die teilweise mit Schwefelkohlenstoff beladene Methylaminlösung kann dann mit Vorteil zur Zubereitung der ersten wässrigen Lösung im erfindungsgemäßen Verfahren verwendet werden. Die Abgaswäsche mit der als Edukt im erfindungsgemäßen Verfahren verwendeten Methylaminlösung kommt vorzugsweise bei einem kontinuierlichen Verfahren zum Einsatz.

Alternativ oder zusätzlich kann man die Abgaswäsche mit einem Alkylendiamin, entweder in Form einer wässrigen Lösung oder - falls das Alkylendiamin bei der Behandlungstemperatur hinreichend niedrig viskos ist - in Substanz, durchführen. Das dabei anfallende, teilweise mit Schwefelkohlenstoff beladene Alkylendiamin wird dann mit Vorteil gemäß einer bevorzugten Ausgestaltung des erfindungsgemäßen Verfahrens zur Zubereitung der Methylammonium-N-methyldithiocarbamat enthaltenden ersten wässrigen Lösung verwendet, wie oben beschrieben.

Spuren von Eisenionen, die z.B. von Korrosion der verwendeten Behälter herrühren oder im Ansatzwasser enthalten sind, können zu unerwünschten Verfärbungen des erhaltenen Tetrahydro-3,5-dimethyl-1,3,5-thiadiazin-2-thions führen. Durch Zusatz von Chelatbildnern, wie Nitrilotriessigsäure, Ethylendiamintetraessigsäure, N-(2-Hydroxyethyl)-ethylendiamintriessigsäure, Diethylentriaminpentaessigsäure, die in Form der freien Säure oder als Alkalimetallsalz, vorzugsweise Natriumsalz eingesetzt werden können, kann eine Farbaufhellung erzielt werden. Der Zusatz des Komplexbildners kann an einer beliebigen Stelle des erfindungsgemäßen Verfahrens bzw. bei der Umsetzung von Methylamin mit Schwefelkohlenstoff erfolgen, vorzugsweise bei der Umsetzung von Schwefelkohlenstoff und Methylamin. Geeignete Mengen sind z.B. 0,05 bis 0,5 Gew.-%, bezogen auf das Gewicht des gebildeten Tetrahydro-3,5-dimethyl-1,3,5-thiadiazin-2-thions.

Die Erfindung wird durch die folgenden Beispiele und Vergleichsbeispiele näher veranschaulicht.

Vergleichsbeispiel A (diskontinuierliche Fällung):

In einer Kaskade aus zwei 4 l-Reaktoren, die über einen barometrischen Austrag miteinander verbunden waren, wurden bei 35°C pro Stunde 2530,1 g Wasser, 2476,8 g 40%-iges wässriges Monomethylamin, 20,7 g Ethylendiamin und 1540,5 g CS₂ umgesetzt. Die erhaltene Suspension wurde durch eine als Koaleszenzfilter betriebene Filterkerze geleitet. Nach Phasentrennung vom überschüssigen CS₂ erhielt man so 6351,8 g Methyl-ammonium-N-methyldithiocarbamat als 36,5%-ige wässrige Lösung (Restgehalt an CS₂ etwa 0,05 Gew.-%) pro Stunde.

In einem 160 l-Reaktor wurden 79,8 kg Wasser, 26,61 kg 40%-iges Formalin und 1,42 kg Impfmaterial (mittlere Teilchengröße < 50 µm) bei 20°C vorgelegt und innerhalb von 2 h insgesamt 61 kg Methylammonium-N-methyldithiocarbamat als 36,5%-ige wässrige Lösung zudosiert. Man erhielt nach Abfiltrieren 27,4 kg Dazomet.

| | | |
|---|---|---|
| Korngrößen | 42,0% | < 100 µm |
| | 98,4% | < 200 µm |
| | 99,6% | < 300 µm |
| | 99,8% | < 400 µm |
| | | |
| Schüttdichte | 0,69 kg/l | |

L-Farbwert (UV-VIS): 63,1 (bestimmt durch Reflexionsmessung, wobei 100 = vollständige Reflexion; 0= vollständige Absorption von Normlicht)

### Beispiel B (kontinuierliche Fällung ohne Animpfen):

In einer Kaskade aus zwei 4 l-Reaktoren, die über einen barometrischen Austrag miteinander verbunden waren, wurden bei 35°C pro Stunde 1686,7 g Wasser, 1651,2 g 40%-iges wässriges Monomethylamin, 11,1 g Ethylendiamin und 1027,0 g CS₂ umgesetzt, wobei das Ethylendiamin in den zweiten Reaktor dosiert wurde. Die Suspension wurde durch eine als Koaleszenzfilter betriebene Filterkerze geleitet. Nach Phasentrennung vom überschüssigen CS₂ erhielt man so 4234,5 g Methylammonium-N-methyldithiocarbamat als 36,5%-ige wässrige Lösung (Restgehalt an CS₂ etwa 0,05 Gew.-%) pro Stunde.

In einen 7 l-Reaktor wurden bei 25°C pro Stunde 2301,2 g Methylammonium-N-methyldithiocarbamat als 36,5%-ige wässrige Lösung, 993,0 g 40%-iges Formalin und 3078,7 g zurückgeführte Mutterlauge dosiert. Durch Zentrifugation wurden 1146,7 g restfeuchtes Dazomet erhalten.

| | | |
|---|---|---|
| Korngrößen | 0,1% | < 100 µm |
| | 25,6% | < 200 µm |
| | 91,6% | < 300 µm |
| | 96,4% | < 400 µm |
| | | |
| Schüttdichte | 0,49 kg/l | |

Die obigen Verbindungen (1), (2) und (3) (mit A = 1,2-Ethylen) sind im Verhältnis 33:34:33 enthalten.

L-Farbwert (UV-VIS): 77,0

Bei Zusatz von 3 g Ethylendiamintetraessigsäure-dinatriumsalz pro Stunde in die Umsetzung von Methylamin und Schwefelkohlenstoff erhielt man Dazomet mit einem Farbwert von 92,3.

### Beispiel C (kontinuierliche Fällung mit Animpfen):

In einem 160 l-Reaktor wurden pro Stunde 57,7 kg Methylammonium-N-methyldithiocarbamat als 36,5%-ige wässrige Lösung, die wie in Beispiel B beschrieben hergestellt worden ist, 28,1 kg 40%-iges Formalin, 83,6 kg Wasser und 1,0 kg Impfmaterial (mittlere Teilchengröße < 50 µm) umgesetzt. Das erhaltene Dazomet wies folgende Eigenschaften auf:

| | | |
|---|---|---|
| Korngrößen | 6,8% | < 100 µm |
| | 73,6% | < 200 µm |
| | 95,6% | < 300 µm |
| | 98,6% | < 400 µm |
| | | |
| Schüttdichte | 0,68 kg/l | |

### Beispiel D (kontinuierliche Fällung mit Animpfen):

In einem 160 l-Reaktor wurden pro Stunde 28,85 kg Methylammonium-N-methyldithiocarbamat als 36,5%-ige wässrige Lösung, die wie in Beispiel B beschrieben hergestellt worden ist, 14,05 kg 40%-iges Formalin, 41,8 kg Wasser und 0,1 kg Impfmaterial (mittlere Teilchengröße < 50 µm) umgesetzt. Das erhaltene Dazomet wies folgende Eigenschaften auf:

| | | |
|---|---|---|
| Korngrößen | 2% | < 100 µm |
| | 50,4% | < 200 µm |
| | 93,2% | < 300 µm |
| | 96,8% | < 400 µm |

## Patentansprüche

1. Verfahren zur Herstellung einer teilchenförmigen Zubereitung von Tetrahydro-3,5-dimethyl-1,3,5-thiadiazin-2-thion durch Vereinigen einer Methylammonium-N-methyldithiocarbamat enthaltenden ersten wässrigen Lösung mit einer Formaldehyd enthaltenden zweiten wässrigen Lösung und Abtrennen und Trocknen des gebildeten Feststoffs, **dadurch gekennzeichnet, dass** man die erste und die zweite wässrige Lösung so vereinigt, dass über die Dauer der Umsetzung das Verhältnis der Konzentrationen von Dithiocarbamat-Funktionen und Formaldehyd im Reaktionsgemisch zeitlich im Wesentlichen konstant ist, und aus der erhaltenen Suspension den Feststoff abtrennt.

2. Verfahren nach Anspruch 1, **dadurch gekennzeichnet, dass** man in einen Reaktionsraum gleichzeitig solche Massenströme der ersten und der zweiten wässrigen Lösung einführt, dass pro Zeiteinheit stöchiometrisch im Wesentlichen äquivalente Mengen an Dithiocarbamat-Funktionen und Formaldehyd eingeführt werden.

3. Verfahren nach Anspruch 1 oder 2, **dadurch gekennzeichnet, dass** man die erste und die zweite wässrige Lösung in Gegenwart von feinverteiltem Tetrahydro-3,5-dimethyl-1,3,5-thiadiazin-2-thion vereinigt.

4. Verfahren nach einem der vorhergehenden Ansprüche, **dadurch gekennzeichnet, dass** die erste wässrige Lösung außerdem ein Alkylendiamin und/oder Umsetzungsprodukte davon mit Schwefelkohlenstoff enthält.

5. Verfahren nach Anspruch 4, **dadurch gekennzeichnet, dass** die erste wässrige Lösung Alkylendiamin, N-Aminoalkyldithiocarbamat und Alkylen-N,N'-bis(dithiocarbamat) in einem molaren Verhältnis von 1:0,5:0,5 bis 1:10:10 enthält.

6. Verfahren nach einem der vorhergehenden Ansprüche, **dadurch gekennzeichnet, dass** man die erste wässrige Lösung erhält, indem man eine wässrige Lösung von Methylamin mit einem stöchiometrischen Überschuß an Schwefelkohlenstoff umsetzt und den nicht umgesetzten Schwefelkohlenstoff auf weniger als 0,5 Gew.-%, bezogen auf die wässrige Lösung, entfernt.

7. Verfahren nach Anspruch 6, **dadurch gekennzeichnet, dass** man die wässrige Lösung zur Entfernung des nicht umgesetzten Schwefelkohlenstoffs durch eine Apparatur mit koaleszenz-fördernden Einbauten leitet und die koaleszierte Schwefelkohlenstoffphase abtrennt.

8. Verfahren nach einem der vorhergehenden Ansprüche, **dadurch gekennzeichnet, dass** man die erste und die zweite wässrige Lösung in Gegenwart eines Chelatbildners für Eisenionen vereinigt.

9. Verfahren nach einem der Ansprüche 6 bis 8, **dadurch gekennzeichnet, dass** man als wässrige Lösung von N-Methylamin eine teilweise mit Schwefelkohlenstoff beladene wässrige Methylaminlösung verwendet, die bei der Wäsche der Schwefelkohlenstoff enthaltenden Abluft des Verfahrens mit einer wässrigen Methylaminlösung erhalten wird.

10. Verfahren nach einem der Ansprüche 4 bis 9, **dadurch gekennzeichnet, dass** man als Alkylendiamin ein teilweise mit Schwefelkohlenstoff beladenes Alkylendiamin verwendet, das bei der Wäsche der Schwefelkohlenstoff enthaltenden Abluft des Verfahrens mit einem Alkylendiamin oder einer wässrigen Lösung davon erhalten wird.

## Claims

1. A process for the preparation of a particulate tetrahydro-3,5-dimethyl-1,3,5-thiadiazine-2-thione product by combining a first aqueous solution comprising methylammonium N-methyldithiocarbamate with a second aqueous solution comprising formaldehyde, followed by separation and drying of the resulting solid, which comprises combining the first and the second aqueous solutions in such a way that the ratio between the concentrations of dithiocarbamate functions and of formaldehyde is essentially constant in the reaction mixture over time during the duration of the reaction, and separating the solid from the resulting suspension.

2. The process as claimed in claim 1, wherein the mass flows of the first and of the second aqueous solution which are introduced simultaneously into the reaction space are such that stoichiometrically essentially equivalent quantities of dithiocarbamate functions and formaldehyde are introduced per unit time.

3. The process as claimed in claim 1 or 2, wherein the first and the second aqueous solutions are combined in the presence of finely divided tetrahydro-3,5-dimethyl-1,3,5-thiadiazine-2-thione.

4. The process as claimed in any of the preceding claims, wherein the first aqueous solution additionally comprises an alkylenediamine and/or reaction products thereof with carbon disulfide.

5. The process as claimed in claim 4, wherein the first aqueous solution comprises alkylenediamine, N-aminoalkyldithiocarbamate and alkylene-N,N'-bis(dithiocarbamate) in a molar ratio of 1:0.5:0.5 to 1:10:10.

6. The process as claimed in any of the preceding claims, wherein the first aqueous solution is obtained by reacting an aqueous solution of methylamine with a stoichiometric excess of carbon disulfide and removing the unreacted carbon disulfide to less than 0.5% by weight based on the aqueous solution.

7. The process as claimed in claim 6, wherein the aqueous solution for removing the unreacted carbon disulfide is passed through an apparatus with coalescence-enhancing elements and the coalesced carbon disulfide is phase separated off.

8. The process as claimed in any of the preceding claims, wherein the first and the second aqueous solutions are combined in the presence of a chelating agent for iron ions.

9. The process as claimed in any of claims 6 to 8, wherein the aqueous N-methylamine solution used is an aqueous methylamine solution loaded with some carbon disulfide, which solution is obtained when the process waste air, which contains carbon disulfide, is scrubbed with an aqueous methylamine solution.

10. The process as claimed in any of claims 4 to 9, wherein the alkylenediamine used is an alkylenediamine loaded with some carbon disulfide, which alkylenediamine is obtained when the process waste air, which contains carbon disulfide, is scrubbed with an alkylenediamine or an aqueous solution thereof.

## Revendications

1. Procédé de production d'une préparation en forme de particules de tétrahydro-3,5-diméthyl-1,3,5-thiadiazine-2-thione par réunion d'une première solution aqueuse contenant du N-méthyldithiocarbamate de méthylammonium avec une seconde solution aqueuse contenant du formaldéhyde, puis séparation et séchage du solide formé, **caractérisé en ce qu'**on rassemble la première et la seconde solution aqueuse de manière que pendant la durée de la réaction le rapport des concentrations de fonctions dithiocarbamate et de formaldéhyde dans le mélange réactionnel soit essentiellement constant dans le temps, et **en ce qu'**on sépare le solide à partir de la suspension obtenue.

2. Procédé selon la revendication 1, **caractérisé en ce qu'**on introduit dans un espace de réaction de façon simultanée des courants massiques de la première et de la seconde solution aqueuse, tels que par unité de temps des quantités stoechiométriquement essentiellement équivalentes de fonctions dithiocarbamate et de formaldéhyde soient introduites.

3. Procédé selon la revendication 1 ou 2, **caractérisé en ce qu'**on rassemble la première et la seconde solution aqueuse en présence de tétrahydro-3,5-diméthyl-1,3,5-thiadiazine-2-thione finement divisée.

4. Procédé selon l'une des revendications précédentes, **caractérisé en ce que** la première solution aqueuse contient en outre une alkylènediamine et/ou ses produits de réaction avec le sulfure de carbone.

5. Procédé selon la revendication 4, **caractérisé en ce que** la première solution aqueuse contient une alkylènediamine, du dithiocarbamate de N-aminoalkyle et un N,N'-bis(dithiocarbamate) d'alkylène dans un rapport molaire de 1 : 0,5 : 0,5 à 1 : 10 : 10.

6. Procédé selon l'une des revendications précédentes, **caractérisé en ce qu'**on obtient la première solution aqueuse en faisant réagir une solution aqueuse de méthylamine avec un excès stoechiométrique de sulfure de carbone et en retirant le sulfure de carbone qui n'a pas réagi pour le ramener à moins de 0,5% par rapport à la solution aqueuse.

7. Procédé selon la revendication 6, **caractérisé en ce qu'**on conduit la solution aqueuse, pour éliminer le sulfure de carbone qui n'a pas réagi, à travers un appareil comportant des insertions favorisant la coalescence, et **en ce qu'**on sépare la phase sulfure de carbone en coalescence.

8. Procédé selon l'une des revendications précédentes, **caractérisé en ce qu'**on rassemble la première et la seconde solution aqueuse en présence d'un agent chélatant pour les ions fer.

9. Procédé selon l'une des revendications 6 à 8, **caractérisé en ce qu'**on utilise comme solution aqueuse de N-méthylamine une solution aqueuse de méthylamine partiellement chargée en sulfure de carbone, que l'on obtient lors du lavage de l'air sortant du procédé, contenant du sulfure de carbone, avec une solution aqueuse de méthylamine.

10. Procédé selon l'une des revendications 4 à 9, **caractérisé en ce qu'**on utilise comme alkylènediamine une alkylènediamine partiellement chargée en sulfure de carbone, que l'on obtient lors du lavage de l'air sortant du procédé, contenant du sulfure de carbone, avec une alkylènediamine ou une solution aqueuse de celle-ci.
